# EUROPEAN PATENT SPECIFICATION

(11) **EP 3 413 938 B1**
(45) Date of publication and mention of the grant of the patent: **10.03.2021**
(21) Application number: 17703609.2
(22) Date of filing: 26.01.2017
(51) Int. Cl.: A61L 27/36, A61L 27/56, A61F 2/12

(54) **BIOLOGIC BREAST IMPLANT**
BIOLOGISCHES BRUSTIMPLANTAT
IMPLANT MAMMAIRE BIOLOGIQUE

(30) Priority: 08.02.2016 US 201662292515 P
(43) Date of publication of application: 19.12.2018
(73) Proprietor: LifeCell Corporation, Madison, New Jersey 07940 (US)
(72) Inventor: SHAH, Mrinal, Parsippany NJ 07054 (US); CHEN, Yi, Lawrenceville NJ 08648 (US); PARK, Sangwook, Dunellen NJ 08812 (US); JESSOP, Israel, Annandale NJ 08801 (US)
(74) Representative: Forresters IP LLP
(86) International application number: PCT/US2017/015011
(87) International publication number: WO 2017/139100

(56) References cited:
- EP-A1- 1 216 718
- WO-A1-2011/046806
- US-A- 4 950 483
- US-A1- 2012 310 367
- US-A1- 2015 037 436

## Description

This application claims priority under 35 U.S.C. § 119 to United States Provisional Application Number 62/292,515, which was filed on February 8, 2016.

The present disclosure relates to tissue products, and more particularly, to extracellular tissue matrices made from adipose tissue.

Various tissue-derived products are used to regenerate, repair, or otherwise treat diseased or damaged tissues and organs. Such products can include tissue grafts and/or processed tissues (e.g., acellular tissue matrices from skin, intestine, or other tissues, with or without cell seeding). Such products generally have properties determined by the tissue source *(i.e.,* tissue type and animal from which it originated) and the processing parameters used to produce the tissue products. Since tissue products are often used for surgical applications and/or tissue replacement or augmentation, the products should support tissue growth and regeneration, as desired for the selected implantation site.

US 2012/310367 describes a method for producing a tissue product, comprising selecting an adipose tissue, reducing the tissue size and treating said tissue particles to remove substantially all cellular material, suspending the resulting particles in a solution which is then placed in a mold to produce a desired shape. This structure is freeze-dried to obtain a porous sponge and cross-linked to produce a stable three-dimensional structure. The tissue product can be used for breast tissue replacement or augmentation. The present disclosure provides adipose tissue products that can allow improved tissue growth and regeneration for various applications, such as breast implants.

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

According to a first embodiment, a method for producing a tissue product according to independent claim 1 is provided. This method includes selecting an adipose tissue; mechanically processing the adipose tissue to reduce the tissue size; and treating the mechanically processed tissue to remove substantially all cellular material from the tissue; suspending the tissue in a liquid to form a suspension; layering the suspension in a mold, wherein the layering is repeated until a desired thickness is achieved in the mold; and freeze-drying the suspension in the mold to form a porous sponge, wherein the sponge includes a first layer having a first density and at least one additional layer having a second density that is different from the first density. The processed tissue can be cross-linked to produce a stable three-dimensional structure.

In some embodiments, the methods include performing the freezing and drying steps after each layering cycle. Alternatively, the methods can include performing the freezing and drying steps after multiple layering cycles, or performing multiple freezing and drying cycles after each layering step.

Also provided herein are tissue products made by the disclosed processes. For example, the tissue products can be made by a process comprising selecting an adipose tissue; treating the tissue to remove substantially all cellular material from the tissue; suspending the tissue in a liquid to form a suspension; layering the suspension in a mold, wherein the layering is repeated in multiple cycles until a desired thickness is achieved in the mold; and freeze-drying the suspension in the mold to form a porous sponge, wherein the two or more layers include a first layer having a first density and at leat one additional layer having a second density that is different than the first density.

In some embodiments, the tissue products can be made by a process that further includes performing the freezing and drying step after each layering cycle. Alternatively, the process further includes performing the freezing and drying step after multiple layering cycles; or performing multiple freezing and drying cycles after each layering step.

In some embodiments, the tissue products include a decellularized adipose extracellular tissue matrix, wherein the tissue matrix has been formed into a predetermined three-dimensional shape, and wherein the tissue matrix is partially cross-linked to maintain the three-dimensional shape.

Also provided herein is a tissue product comprising a breast implant, as defined in independent claim 10. The implant comprises a porous sponge formed of a layered construct of acellular adipose tissue matrix including two or more layers of particulate acellular adipose tissue matrix that has been homogenized to form a suspension, dried, and stabilized. The implant measures at least 5 cm in at least one dimension, and the two or more layers include a first layer having a first density and at least one additional layer having a second density that is different than the first density.

Also described herein, but not part of the claimed invention, are methods of treatment comprising the steps of selecting a tissue site and implanting the tissue products disclosed herein into the tissue site. The methods can include implanting the treatment device in or proximate a wound or surgical site and securing at least a portion of the treatment device to tissue in or near the treatment site. The tissue product may be implanted behind the tissue site to bolster, reposition, or project the native tissue outward.

Also described herein, but not part of the claimed invention, are methods of treatment comprising selecting a tissue site within a breast; implanting a device within the tissue site. In one embodiment, the device comprises a synthetic breast implant or tissue expander and an acellular adipose tissue matrix surrounding the breast implant or tissue expander. The method can further include removing the breast implant or tissue expander and implanting an acellular adipose tissue matrix within a void formed by removal of the breast implant or tissue expander.

Additionally, described herein, but not part of the claimed invention, are breast treatment devices comprising a synthetic breast implant or tissue expander and a layered construct of acellular adipose tissue matrix surrounding the synthetic breast implant or tissue expander. In one embodiment, the construct includes two or more layers of particulate acellular adipose tissue matrix that has been homogenized to form a suspension, dried, and stabilized.

### BRIEF DESCRIPTION OF THE DRAWINGS

Fig. 1 is a flow chart illustrating a process for producing adipose tissue products, according to certain embodiments.
Fig. 2 is a side view of a biologic breast implant having a layered construct, according to certain embodiments.
Fig. 3A is a perspective view of a configuration for a breast implant, having a layered construct, according to certain embodiments.
Fig. 3B is a perspective view of another configuration for a breast implant, having a layered construct, according to certain embodiments.
Fig. 3C is a perspective view of another configuration for a breast implant, having a layered construct, according to certain embodiments.
Fig. 4 is a side view of another breast implant including a synthetic implant or tissue expander and an adipose tissue matrix coating, according to certain embodiments.
Fig. 5 is a scanning electron microscope image of an adipose tissue product having a layered construct produced according to various embodiments.
Fig. 6 illustrates implantation of a system for surgical breast procedures, including a pre-shaped tissue matrix, according to certain embodiments.
Fig. 7A illustrates implantation of a system for surgical breast procedures, including a breast implant or tissue expander surrounded by an adipose tissue matrix coating, according to certain embodiments.
Fig. 7B illustrates a second step in a treatment process using the implant illustrated in Fig. 7A, wherein the breast implant or tissue expander is replaced with a biologic breast implant, according to certain embodiments.

### DESCRIPTION OF CERTAIN EXEMPLARY EMBODIMENTS

Reference will now be made in detail to certain exemplary embodiments according to the present disclosure, certain examples of which are illustrated in the accompanying drawings. Wherever possible, the same reference numbers will be used throughout the drawings to refer to the same or like parts.

In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Any range described herein will be understood to include the endpoints and all values between the endpoints.

The section headings used herein are for organizational purposes only and are not to be construed as limiting the subject matter described.

Various human and animal tissues can be used to produce products for treating patients. For example, various tissue products for regeneration, repair, augmentation, reinforcement, and/or treatment of human tissues that have been damaged or lost due to various diseases and/or structural damage (e.g., from trauma, surgery, atrophy, and/or long-term wear and degeneration) have been produced. Such products can include, for example, acellular tissue matrices, tissue allografts or xenografts, and/or reconstituted tissues *(i.e.,* at least partially decellularized tissues that have been seeded with cells to produce viable materials).

The present disclosure provides tissue products that are useful for treatment of adipose-containing tissues as wells as other tissue sites, including breasts. The present disclosure also provides methods for producing such tissue products.

The tissue products of the present disclosure can include adipose tissues that have been processed to remove at least some of the cellular components. In some cases, all, or substantially all cellular material is removed, thereby leaving adipose extracellular matrix proteins. In addition, the products can be processed to remove some (e.g., 10-20%, 20-30%, 30-40%, 40-50%, 60-70%, 70-80%, 80-90%, 70-90%, 70-95% (all by weight)) or all of the extracellular and/or intracellular lipids. As described further below, the extracellular matrix proteins can be further treated to produce a three-dimensional porous, or sponge-like material. In addition, to allow treatment of a selected tissue site the material can be further processed (e.g., by cross-linking) to form a stable structure.

The tissue products of the present disclosure can be used as breast implants, e.g., for breast augmentation, reconstruction (e.g., after mastectomy or other breast surgery), or any other breast procedure. The breast implants comprise a layered construct of acellular adipose tissue matrix. In one embodiment, a layered construct of acellular adipose tissue matrix comprises two or more layers. The implant measures at least 5 cm in at least one dimension, and the two or more layers include a first layer having a first density and at least one additional layer having a second density that is different than the first density.

The layered construct of such breast implants is achieved through a layering process in which each layer comprises particulate acellular adipose tissue that has been mechanically processed to form a suspension, dried, and stabilized. This layering process allows for the production of an implant that is big enough to act as a breast implant *(i.e.,* an implant that is dimensionally sufficient to replace all or part of a breast).

As noted, the tissue products of the present disclosure are formed from adipose tissues. The adipose tissues can be derived from human or animal sources. For example, human adipose tissue can be obtained from cadavers. In addition, human adipose tissue could be obtained from live donors *(e.g.,* with an autologous tissue). Adipose tissue may also be obtained from animals such as pigs, monkeys, or other sources. If animal sources are used, the tissues may be further treated to remove antigenic components such as 1,3-alpha-galactose moieties, which are present in pigs, but not humans or primates. In addition, the adipose tissue can be obtained from animals that have been genetically modified to remove antigenic moieties. *See* Xu, Hui, et al., "A Porcine-Derived Acellular Dermal Scaffold that Supports Soft Tissue Regeneration: Removal of Terminal Galactose-α-(1,3)-Galactose and Retention of Matrix Structure," Tissue Engineering, Vol. 15, 1-13 (2009).

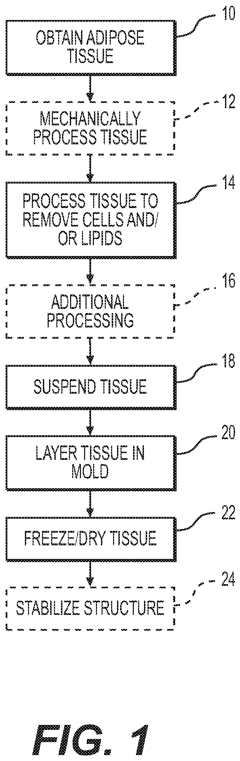

An exemplary process for producing the tissue products of the present disclosure is illustrated in Fig. 1. As shown, the process generally includes obtaining adipose tissue (Step **10);** processing the tissue to remove substantially all cellular material and/or lipids from the tissue (Step **14)** (as noted, partial lipid removal may be desired e.g., 10-20%, 20-30%, 30-40%, 40-50%, 60-70%, 70-80%, 80-90%, 70-90%, 70-95% (all by weight))); suspending the tissue in a liquid (Step **18);** layering the tissue in a mold (Step **20);** and freezing and drying the suspension in the mold to form a porous sponge (Step **22).** The process optionally includes one or more of mechanically processing the adipose tissue to produce small pieces (Step **12);** additional processing of the tissue (Step **16);** and stabilizing (e.g., for example, but not necessarily by cross-linking) the tissue to produce a stable three-dimensional structure (Step **24).** Each of these steps is explained in more detail below.

To assist in removal of the cellular components and produce a flowable mass, the tissue can be first processed to produce small pieces (Step **12).** In various embodiments, the material is cut, grinded, blended, or otherwise mechanically treated to reduce the size of the tissue and/or to form a putty or flowable material. The adipose tissue can be treated using any suitable cutting, grinding, or blending process. For example, in one embodiment, the tissue is first cut into relatively small pieces *(e.g.,* about 2 cmx2 cm). The pieces can then be placed in a liquid that is then treated with a blade grinder or similar instrument to produce a homogenous or semi-homogenous material.

Next, the tissue is treated to remove cellular components and/or lipids. The cellular components and lipids can be removed by washing the material (Step **14).** For example, in some embodiments, the material is combined with a desired amount of water or another solvent. The material and solvent are then centrifuged, and free lipids and cell debris will flow to the top, while the extracellular matrix proteins are deposited as a pellet. The protein pellet can then be resuspended, and the washing and centrifugation can be repeated until a sufficient amount of the lipids and cellular materials are removed. In some cases, the process is repeated until substantially all cellular material are removed and until a desired amount of lipid is removed.

During, before, and/or after the washing steps, additional solutions or reagents can be used to process the material (Step **16).** For example, enzymes, detergents, and/or other agents may be used in one or more steps to remove cellular materials or lipids, remove antigenic materials, and/or reduce the bacteria or other bioburden of the material. For example, one or more washing steps can be included using detergents to assist in cell and lipid removal. In addition, enzymes such as lipases, DNAses, RNAses, alpha-galactosidase, or other enzymes can be used to ensure destruction of nuclear materials, antigens from xenogenic sources, and/or viruses.

After removal of cellular components, the material can then be formed into a porous or sponge-like material. Generally, the extracellular matrix is first suspended in an aqueous solvent (Step **18).** A sufficient amount of solvent is used to allow the material to form a liquid mass that can be poured into a mold having the size and shape of the desired tissue product. The amount of water added can be varied based on the desired porosity of the final material. In some cases, the suspended extracellular matrix may be mechanically treated by grinding, cutting, blending or other processes one or more additional times, and the treated material can be centrifuged and resuspended one or more times to further remove cellular material or lipids (if needed) and/or to control the viscosity of the extracellular matrix.

Once any washing and grinding steps are complete, the suspended material is placed in a container or mold to form the porous, sponge-like product by a layering process (Step **20).** In one embodiment, the layering process is repeated until a desired thickness is achieved in the mold. In some embodiments, the desired thickness is at least or exceeds 1.0 cm, 3.0 cm, 5.0 cm, 7.0 cm, 9.0 cm, 11.0 cm, 13.0 cm, or 15.0 cm.

According to the claimed invention, the porous or sponge-like material is formed by freeze-drying the material to leave a three-dimensional matrix with a porous structure (Step **22).** Freeze-drying can allow production of a three-dimensional structure that generally conforms to the shape of the mold.

The specific freeze drying protocol can be varied based on the solvent used, sample size, and/or to optimize processing time. One suitable freeze-drying process can include freezing the material to a temperature of -10° C to - 20° C over a 30 to 45 minute period; further cooling the material down to a temperature of -40° C to -60° C to ensure complete freezing of all bound and unbound water in the sample; applying a vacuum of 13.3 to 33.3 Pa (100 to 250 mTorr); raising the temperature to -10° C to -5° C and holding at this condition until primary drying is completed; further raising the temperature to 20° C for secondary drying and holding for 3 to 12 hours. The freeze-dried samples can then be removed from the freeze-dryer and packaged under a nitrogen blanket in moisture barrier pouches, such as foil pouches. An alternative freeze drying cycle for thicker cross-sections (8 cm or larger) includes a longer duration for primary drying (-10°C hold for 72-192 hours), followed by secondary drying at 20°C for 24 - 48 hours. A third freeze drying strategy is the application of several (2-10) nitrogen purge or deep vacuum cycles to facilitate heat transfer into the tissue during primary drying. A fourth strategy is the use of microwave-assisted freeze drying to impart thermal energy to the water or ice during primary drying. This accelerates heat transfer and sublimation of water from the matrix.

In some embodiments, the methods include performing the freezing and drying step after each layering cycle. Alternatively, the methods include performing the freezing and drying step after multiple layering cycles; or performing multiple freezing and drying cycles after each layering step.

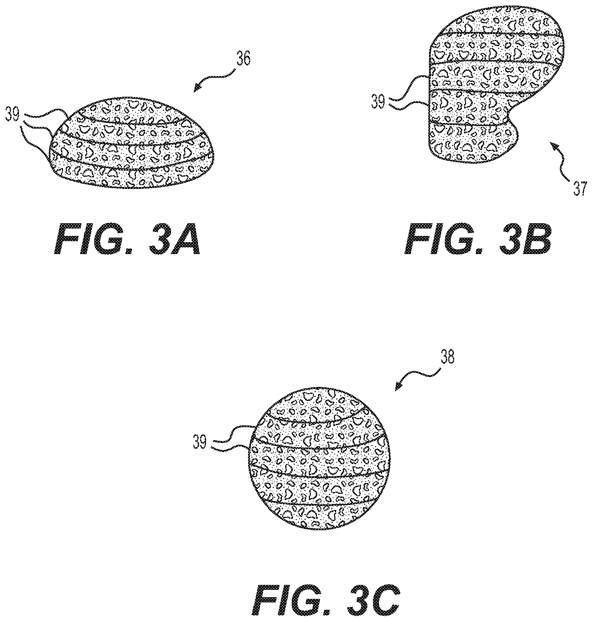

After forming the porous, sponge-like material, the material can be treated so that it forms a stable three-dimensional shape (Fig. 3). For example, the mechanically processed tissue, when formed into a porous matrix, may form a putty- or paste-like material when it is implanted in a body, becomes wet, or is placed in a solution. Therefore, the desired shape and size may be lost. In addition, the porous structure, which may be important for supporting cell attachment, tissue growth, vascular formation, and tissue regeneration, may be lost. Accordingly, the material should be further processed to stabilize the size, shape, and structure of the material.

In some embodiments, the material is cross-linked to perform the stabilization (Step **24).** In some embodiments, the material is cross-linked after freeze drying. However, the material could also be cross-linked before or during the freeze-drying process. Cross-linking can be performed in a variety of ways. In one embodiment, cross-linking is accomplished by contacting the material with a cross-linking agent such as glutaraldehyde, genipin, carbodiimides, and diisocyanates. In addition, cross-linking can be performed by heating the material. For example, in some embodiments, the material can be heated to between 70° C to 120° C, or between 80° C and 110° C, or to about 100° C, or any values between the specified ranges in a reduced pressure or vacuum or dry gas. Further, the cross-linking can be performed by dehydrothermal treatment, including heating in a reduced pressure environment to remove moisture.

In addition, other cross-linking processes may be used to produce any of the disclosed products, including ultraviolet irradiation, gamma irradiation, and/or electron beam irradiation. In addition, a vacuum is not needed but may reduce cross-linking time. Further, lower or higher temperatures could be used as long as melting of the matrix proteins does not occur and/or sufficient time is provided for cross-linking.

In various embodiments, the cross-linking process can be controlled to produce a tissue product with desired mechanical, biological, and/or structural features. For example, cross-linking may influence the overall strength of the material, and the process can be controlled to produce a desired strength. In addition, the amount of cross-linking can affect the ability of the product to maintain a desired shape and structure *(e.g.,* porosity) when implanted. Accordingly, the amount of cross-linking can be selected to produce a stable three-dimensional shape when implanted in a body, when contacted with an aqueous environment, and/or when compressed *(e.g.,* by surrounding tissues or materials).

Excessive cross-linking may change the extracellular matrix materials. For example, excessive cross-linking may damage collagen or other extracellular matrix proteins. The damaged proteins may not support tissue regeneration when the tissue products are placed in an adipose tissue site or other anatomic location. In addition, excessive cross-linking can cause the material to be brittle or weak. Accordingly, the amount of cross-linking may be controlled to produce a desired level of stability, while maintaining desired biological, mechanical, and/or structural features.

Exemplary cross-linking processes can include contacting a freeze-dried material, produced as discussed above, with glutaraldehyde. For example, a 0.1% glutaraldehyde solution can be used, and the tissue can be submerged in the solution for about for 18 hours followed by extensive rinsing in water to remove the solution. Alternatively, or in combination, a dehydrothermal process can be used. For example, one exemplary dehydrothermal process includes treating the material at about 100° C and about 20 inches of Hg for 18 hours, followed by submersion in water. The final cross-linked tissue products can be stored in a film pouch.

The adipose tissue can be produced as generally described US Patent Publication Number 2012/0310367A1 (Application number US 13/483,674, filed May 30, 2012, to Connor). Such adipose materials can be formed generally by mechanical homogenization, washing, resuspension, and stabilization of the material. The material may be dried (e.g. by freeze drying before or after stabilization), and stabilization can be by dehydrothermal treatment, cross-linking (UV, radiation, or chemical cross-linking). In addition, the sponge may be sterilized. Sterilization may be performed after the components of the devices described herein are joined. Further, the sponge may be formed while in contact with the intact acellular tissue matrix components, or may be formed separately prior to joining. As noted above, the process described is said application can be repeated in layers to produce a desired size, shape, and thickness.

As discussed above, the tissue products should have the ability to support cell ingrowth and tissue regeneration when implanted in or on a patient. In addition, the tissue products should have the ability to act as a carrier for and support the growth of cells, including stems cell, such as adipose-derived stem cells. Accordingly, the processes discussed above should not alter the extracellular matrix proteins (e.g., by damaging protein structure and/or removing important glycosaminoglycans and/or growth factors). In some embodiments, the products will have normal collagen banding as evidenced by transmission electron microscopy.

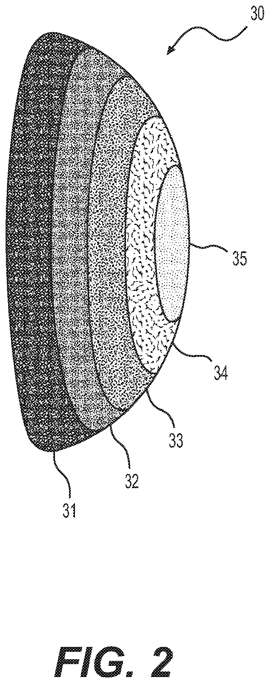

The devices produced using the above-discussed methods can have a variety of configurations. For example, Fig. 2 is a side view of a biologic breast implant 30 having a layered construct according to certain embodiments. The implant 30 can include two or more layers 31-35 of particulate acellular adipose tissue matrix that have been homogenized to form a suspension, dried, and stabilized.

**As** shown, the device 30 can include a number of layers. For example, the implant 30 can include five layers, as shown, but could include a range of layers (e.g., between 1 and 100 layers) depending on the desired size, shape, and functional properties of the implant 30, and depending on the thickness of each layer 31-35.

The various layers 31-35 can have a number of properties that can be varied among the layers 31-35. For example, in one reference-embodiment not according to the claimed invention, each of the layers is substantially identical in mechanical, microscopic, and/or biological properties, but multiple layers are provided to obtain the desired size of an implant 30.

In other embodiments, one or more physical and/or biological properties is varied among or within one or more of the layers. According to the claimed invention the implant is formed from at least two layers, whereby the density of the first layer is different from the density of the second layer. For example, in one embodiment, the layers 31-35 further have variable mechanical or physical properties such as tensile strength, compressibility, pore size, elasticity, or other suitable properties. In addition, the layers 31-35 can include variations in biologic properties, including for example, collagenase susceptibility and/or ability to support or speed of cellular ingrowth.

In one embodiment, one or more mechanical/physical and/or biologic properties can vary from one layer 31 toward an outer layer 35. For example, in one embodiment, the most inner layer when implanted 31 will have the highest tensile strength, to support load bearing, while the most outer layer will have the lowest tensile strength.

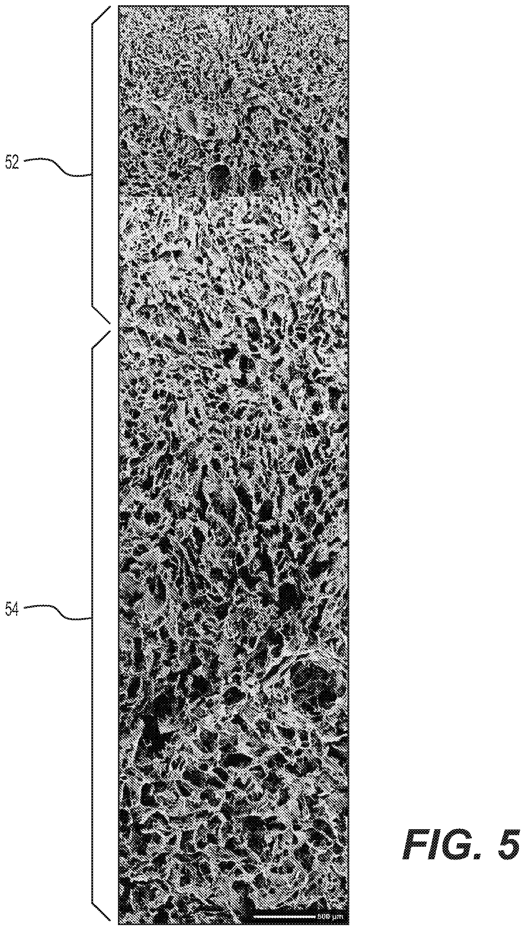

The mechanical/physical and/or biologic properties of the layers 31-35 can be controlled by controlling the processing conditions discussed above, thereby producing variation in density, and optionally also in pore size, collagenase susceptibility or other properties among the layers 31-35. For example, Fig. 5 is a scanning electron microscope image of an adipose tissue product having a layered construct produced according to various embodiments. As shown the material in Fig. 5 has two layers 52 and 54, and the outer layer 54 has a less dense structure (larger tissue matrix pores).

The density of the layers 52 and 54 can be controlled in a number of ways. For example, the density may be controlled by varying the solid content of the slurry used to produce the sponge prior to drying. Suitable solid contents may include for example, between 1% and 20%, or more preferably between 1% and 5%, 1% and 10%, 1% and 15%, 2% and 5%, 2% and 3%, or values within said ranges.

The device 30 shown in Fig. 2 includes a tear-drop shaped breast implant. However, it should be appreciated that a variety of shapes can be used, including rounded, irregular, concentric spheroid or concentric irregular 3-D shapes, or custom-formed implants. For example, Figs. 3A-3C illustrate exemplary shapes for implants produced using the disclosed methods, including tear-drop implants 36 (Fig. 3A), irregular implants 37 (Fig. 3B), and/or spherical implants 38 (Fig. 3C), each formed of layers 39.

The device 30, 36-38 can have a variety of sizes. But as noted above, the methods provided herein can provide advantages by allowing production of adipose implants having large sizes that can match those of conventional breast implants or tissue expanders. According to the claimed invention, using the layering methods discussed herein, implants having at least one dimension of 5 cm or greater are produced. In other cases, the devices have a dimension of at least 6 cm, at least 7 cm, at least 8 cm, at least 10 cm, or larger.

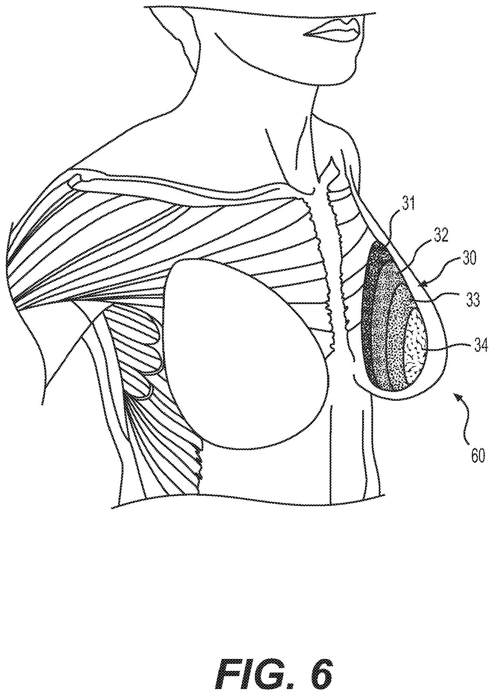

Also disclosed herein, but not part of the claimed invention, are methods for treating a breast by implanting the tissue product. Accordingly, Fig. 6 illustrates implantation of a system for surgical breast procedures, including a pre-shaped tissue matrix implanted with a breast implant or tissue-expander, according to certain embodiments. The method can first include identifying an anatomic site within a breast 30. (As used herein, "within a breast" will be understood to be within mammary tissue, or within or near tissue surrounding the breast such as tissue just below, lateral or medial to the breast, or beneath surrounding tissues including, for example, under chest (pectoralis) muscles 60, and will also include implantation in a site in which part or all of the breast has already been removed via surgical procedure). The site can include, for example, any suitable site needing reconstruction, repair, augmentation, or treatment. Such sites may include sites in which surgical oncology procedures (mastectomy, lumpectomy) have been performed, sites where aesthetic procedures are performed (augmentation or revisions augmentation), or sites needing treatment due to disease or trauma.

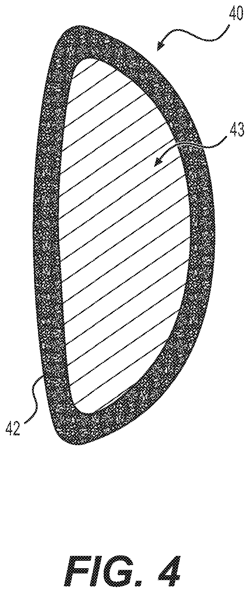

In some reference-embodiments, the layered adipose materials discussed herein can be used along with a synthetic implant or tissue expander. For example, Fig. 4 is a side view of another breast implant 40 including a synthetic implant or tissue expander 43 and an adipose tissue matrix coating 42 having a layered construct according to certain embodiments. As shown, the device includes a conventional or custom-made implant 43, such as a silicone or saline-filled implant, but any synthetic implant can be used.

The synthetic implant or expander 43 can be coated with the adipose tissue matrix 42 and implanted. As such, the tissue matrix can shield the implant or expander 43 from the body to some extent, thereby preventing formation of or improving the quality of fibrotic tissue that may usually form around synthetics. Additionally, or alternatively, the coating 42 can facilitate ingrowth of cells and formation of vascularized tissues, thereby speeding or otherwise improving regeneration of healthy tissue to improve surgical results (e.g., by strengthening surrounding tissue or providing better tissue vascularity).

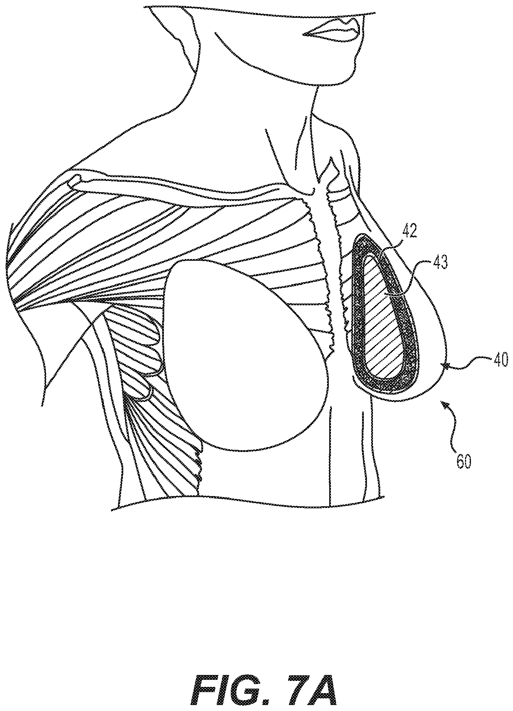

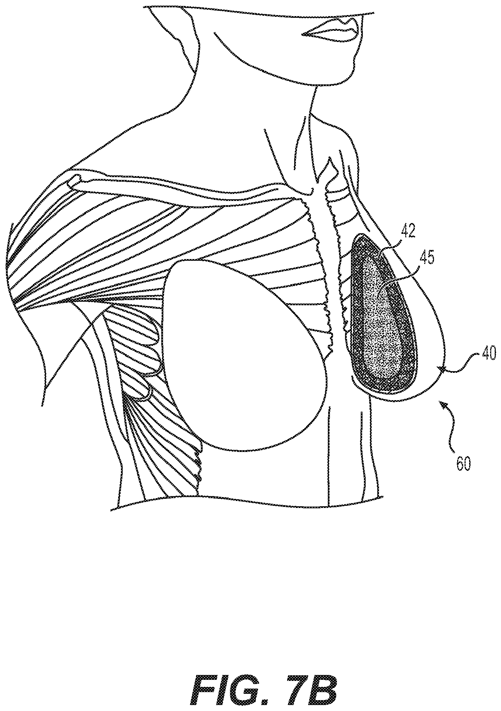

In some cases, the device 40 of Fig. 4 is implanted within a breast site 60, and the implant 43 and coating 42 are left in place (see Fig. 7A). Alternatively, after growth of tissue within the coating 42, the implant or expander 43 can be removed (see Fig. 7B). Then, the void space formed upon removal of the implant or expander 43 can be filled with a biologic material 45, including an acellular tissue matrix. In one embodiment, the material 45 includes a layered adipose material, as discussed above. And as such, the final implant 40 includes a biologic coating 42 and core 45, both of which allow cellular ingrowth and tissue regeneration.

Further described herein, but not part of the claimed invention, are methods of treatment comprising the steps of selecting a tissue site and implanting the tissue products disclosed herein into the tissue site. The methods can include implanting the treatment device in or proximate to a wound or surgical site and securing at least a portion of the treatment device to tissue in or near the treatment site. The tissue product may implanted behind the tissue site to bolster, reposition, or project the native tissue outward.

Also provided herein, but not part of the claimed invention, are methods of treatment comprising selecting a tissue site within a breast; implanting a device within the tissue site, and allowing tissue to grow within the acellular adipose tissue matrix. In one embodiment, the device comprises a synthetic breast implant or tissue expander and an acellular adipose tissue matrix surrounding the breast implant or tissue expander. The method can further include removing the breast implant or tissue expander and implanting an additional acellular adipose tissue matrix within a void formed by removal of the breast implant or tissue expander.

Additionally, described herein, but not part of the claimed invention, are breast treatment devices comprising a synthetic breast implant or tissue expander, and a layered construct of acellular adipose tissue matrix surrounding the synthetic breast implant or tissue expander. In one embodiment, the construct includes two or more layers of particulate acellular adipose tissue matrix that have been homogenized to form a suspension, dried, and stabilized.

After selection of the site, a treatment device is selected. Various devices including acellular tissue matrices can be used, and the devices can include a flexible sheet having a top surface, a bottom surface, and a peripheral border. The peripheral border and shape of the devices can include any configuration discussed herein.

The tissue products described herein can be used to treat a variety of different anatomic sites. For example, as discussed throughout, the tissue products of the present disclosure are produced from adipose tissue matrices and can be used for treatment of breasts. In some cases, the tissue products can be implanted in other sites, including, for example, tissue sites that are predominantly or significantly adipose tissue. In some cases, the tissue sites can include a breast (e.g., for augmentation, replacement of resected tissue, or placement around an implant). In addition, any other adipose-tissue containing site can be selected. For example, the tissue products may be used for reconstructive or cosmetic use in the breast, face, buttocks, abdomen, hips, thighs, or any other site where additional adipose tissue having structure and feel that approximates native adipose may be desired. In any of those sites, the tissue may be used to reduce or eliminate wrinkles, sagging, or undesired shapes.

## Claims

1. A method for producing a tissue product, comprising the steps of:
selecting an adipose tissue;
treating the tissue to remove substantially all cellular material from the tissue;
suspending the tissue in a liquid to form a suspension;
layering the suspension in a mold, wherein the layering is repeated in multiple cycles until a desired thickness is achieved in the mold; and
freeze-drying the suspension to form a porous sponge, wherein the sponge includes a first layer having a first density and at least one additional layer having a second density that is different from the first density.

2. The method of claim 1, further comprising performing the freeze-drying step after each layering cycle.

3. The method of claim 1, further comprising performing the freeze-drying step after multiple layering cycles.

4. The method of claim 1, further comprising performing multiple freeze-drying cycles after each layering step.

5. The method of claim 1, wherein the desired thickness at least in the thickest part of the sponge exceeds 10.0cm.

6. The method of any one of claims 1-5, wherein the mold is in the shape of a round or tear-drop breast implant.

7. The method of any one of claims 1-6, further comprising cross-linking the porous sponge to produce a stable three-dimensional structure.

8. The method of claim 7, wherein cross-linking includes contacting the porous sponge with a cross-linking agent, the cross-linking agent including at least one of glutaraldehyde, genipin, carbodiimides, and diisocyanates.

9. The method of claim 7, wherein cross-linking includes heating the porous sponge, wherein the porous sponge is heated in a vacuum, wherein the porous sponge is heated to 70°C to 120°C.

10. A tissue product, comprising:
a breast implant, the implant comprising a porous sponge formed of a layered construct of acellular adipose tissue matrix including two or more layers of particulate acellular adipose tissue matrix that has been homogenized to form a suspension, dried, and stabilized, and wherein the implant measures at least 5 cm in at least one dimension and wherein the two or more layers include a first layer having a first density and at least one additional layer having a second density that is different than the first density.

11. The tissue product of claim 10, wherein the implant comprises at least five layers of particulate acellular adipose tissue matrix that has been homogenized to form a suspension, dried, and stabilized.

12. The tissue product of any one of claims 10-11, wherein the implant measures at least 8 cm in at least one dimension and/or wherein the implant is in the form of a rounded or tear-drop shaped breast implant.

13. The tissue product of any one of claims 10-12, wherein the implant comprises at least three layers of acellular adipose tissue matrix each having different densities, or wherein the implant comprises at least five layers of acellular adipose tissue matrix each having different densities.

14. The tissue product of claim 13, wherein the density of the layers decreases from one end of the implant to a second end of the implant.

15. The tissue product of any one of claims 13-14, wherein each layer has a difference in at least one of elasticity, tensile strength, or compressive strength.

## Patentansprüche

1. Verfahren zur Herstellung eines Gewebeprodukts, umfassend die folgenden Schritte:
Auswählen eines Fettgewebes;
Behandeln des Gewebes, um im Wesentlichen das gesamte zelluläre Material aus dem Gewebe zu entfernen;
Suspendieren des Gewebes in einer Flüssigkeit, um eine Suspension zu bilden;
Schichtung der Suspension in einer Form, wobei die Schichtung in multiplen Zyklen wiederholt wird, bis eine gewünschte Dicke in der Form erzielt wird; und
Gefriertrocknen der Suspension, um einen porösen Schwamm zu bilden, wobei der Schwamm eine erste Schicht mit einer ersten Dichte und mindestens eine zusätzliche Schicht mit einer zweiten Dichte, die sich von der ersten Dichte unterscheidet, beinhaltet.

2. Verfahren nach Anspruch 1, ferner umfassend das Durchführen des Gefriertrocknungsschritts nach jedem Schichtungszyklus.

3. Verfahren nach Anspruch 1, ferner umfassend das Durchführen des Gefriertrocknungsschritts nach multiplen Schichtungszyklen.

4. Verfahren nach Anspruch 1, ferner umfassend das Durchführen multipler Gefriertrocknungszyklen nach jedem Schichtungsschritt.

5. Verfahren nach Anspruch 1, wobei die gewünschte Dicke mindestens in dem dicksten Teil des Schwamms 10,0 cm überschreitet.

6. Verfahren nach einem der Ansprüche 1-5, wobei die Form in Gestalt eines runden oder tränenförmigen Brustimplantats vorliegt.

7. Verfahren nach einem der Ansprüche 1-6, ferner umfassend das Vernetzen des porösen Schwamms, um eine stabile dreidimensionale Struktur zu erzeugen.

8. Verfahren nach Anspruch 7, wobei das Vernetzen das Inkontaktbringen des porösen Schwamms mit einem Vernetzungsmittel beinhaltet, wobei das Vernetzungsmittel mindestens eines von Glutaraldehyd, Genipin, Carbodiimiden und Diisocyanaten beinhaltet.

9. Verfahren nach Anspruch 7, wobei das Vernetzen das Erhitzen des porösen Schwamms beinhaltet, wobei der poröse Schwamm in einem Vakuum erhitzt wird, wobei der poröse Schwamm auf 70 °C bis 120 °C erhitzt wird.

10. Gewebeprodukt, umfassend:
ein Brustimplantat, wobei das Implantat einen porösen Schwamm umfasst, der aus einem Schichtkonstrukt aus azellulärer Fettgewebematrix beinhaltend zwei oder mehr Schichten partikulärer azellulärer Fettgewebematrix gebildet ist, die homogenisiert, um eine Suspension zu bilden, getrocknet und stabilisiert wurde, und wobei das Implantat mindestens 5 cm in mindestens einer Dimension misst und wobei die zwei oder mehr Schichten eine erste Schicht mit einer ersten Dichte und mindestens eine zusätzliche Schicht mit einer zweiten Dichte, die sich von der ersten Dichte unterscheidet, beinhalten.

11. Gewebeprodukt nach Anspruch 10, wobei das Implantat mindestens fünf Schichten partikulärer azellulärer Fettgewebematrix umfasst, die homogenisiert, um eine Suspension zu bilden, getrocknet und stabilisiert wurde.

12. Gewebeprodukt nach einem der Ansprüche 10-11, wobei das Implantat mindestens 8 cm in mindestens einer Dimension misst und/oder wobei das Implantat in Form eines abgerundeten oder tränenförmigen Brustimplantats vorliegt.

13. Gewebeprodukt nach einem der Ansprüche 10-12, wobei das Implantat mindestens drei Schichten azellulärer Fettgewebematrix, die jeweils verschiedene Dichten aufweisen, umfasst,
wobei das Implantat mindestens fünf Schichten azellulärer Fettgewebematrix, die jeweils verschiedene Dichten aufweisen, umfasst.

14. Gewebeprodukt nach Anspruch 13, wobei die Dichte der Schichten von einem Ende des Implantats zu einem zweiten Ende des Implantats abnimmt.

15. Gewebeprodukt nach einem der Ansprüche 13-14, wobei jede Schicht eine Differenz bei mindestens einer von Elastizität, Zugfestigkeit oder Druckfestigkeit aufweist.

## Revendications

1. Procédé de production d'un produit tissulaire, comprenant les étapes consistant à :
sélectionner un tissu adipeux ;
traiter le tissu pour enlever sensiblement tout le matériel cellulaire du tissu ;
mettre le tissu en suspension dans un liquide pour former une suspension ;
mettre la suspension en couches dans un moule, la mise en couches étant répétée en plusieurs cycles jusqu'à ce qu'une épaisseur souhaitée soit obtenue dans le moule ; et
lyophiliser la suspension pour former une éponge poreuse, l'éponge comprenant une première couche ayant une première densité et au moins une couche supplémentaire ayant une deuxième densité qui est différente de la première densité.

2. Procédé selon la revendication 1, comprenant en outre l'exécution de l'étape de lyophilisation après chaque cycle de mise en couches.

3. Procédé selon la revendication 1, comprenant en outre l'exécution de l'étape de lyophilisation après plusieurs cycles de mise en couches.

4. Procédé selon la revendication 1, comprenant en outre l'exécution de plusieurs cycles de lyophilisation après chaque étape de mise en couches.

5. Procédé selon la revendication 1, l'épaisseur souhaitée, au moins dans la partie la plus épaisse de l'éponge, dépassant 10,0 cm.

6. Procédé selon l'une quelconque des revendications 1 à 5, le moule étant sous la forme d'un implant mammaire rond ou en forme de goutte d'eau.

7. Procédé selon l'une quelconque des revendications 1 à 6, comprenant en outre la réticulation de l'éponge poreuse pour produire une structure tridimensionnelle stable.

8. Procédé selon la revendication 7, la réticulation faisant intervenir la mise en contact de l'éponge poreuse avec un agent de réticulation, l'agent de réticulation renfermant au moins l'un des éléments suivants : glutaraldéhyde, génipine, carbodiimides et diisocyanates.

9. Procédé selon la revendication 7, la réticulation faisant intervenir le chauffage de l'éponge poreuse, l'éponge poreuse étant chauffée sous vide, l'éponge poreuse étant chauffée à une température comprise entre 70°C et 120°C.

10. Produit tissulaire, comprenant :
un implant mammaire, l'implant comprenant une éponge poreuse formée d'une construction en couches consistant en une matrice de tissu adipeux acellulaire renfermant deux couches ou plus de matrice de tissu adipeux acellulaire particulaire qui a été homogénéisé pour former une suspension, séché et stabilisé, l'implant mesurant au moins 5 cm dans au moins une dimension et les deux couches ou plus comprenant une première couche ayant une première densité et au moins une couche supplémentaire ayant une deuxième densité qui est différente de la première densité.

11. Produit tissulaire selon la revendication 10, l'implant comprenant au moins cinq couches de matrice de tissu adipeux acellulaire particulaire qui a été homogénéisé pour former une suspension, séché et stabilisé.

12. Produit tissulaire selon l'une quelconque des revendications 10 à 11, l'implant mesurant au moins 8 cm dans au moins une dimension et/ou l'implant étant sous la forme d'un implant mammaire arrondi ou en forme de goutte d'eau.

13. Produit tissulaire selon l'une quelconque des revendications 10 à 12, l'implant comprenant au moins trois couches de matrice de tissu adipeux acellulaire ayant chacune une densité différente,
l'implant comprenant au moins cinq couches de matrice de tissu adipeux acellulaire ayant chacune une densité différente.

14. Produit tissulaire selon la revendication 13, la densité des couches diminuant d'une extrémité de l'implant à une deuxième extrémité de l'implant.

15. Produit tissulaire selon l'une quelconque des revendications 13 à 14, chaque couche présentant une différence au niveau d'au moins une des caractéristiques suivantes : élasticité, résistance à la traction ou résistance à la compression.
